# EUROPEAN PATENT APPLICATION

(11) **EP 0 642 793 A1**
(43) Date of publication of application: **15.03.1995**
(21) Application number: 93911988.9
(22) Date of filing: 11.05.1993
(51) Int. Cl.: A61K 31/35, C07D 311/30

(54) **APOPTOSIS INDUCER**

(30) Priority: 11.05.1992 JP 143681/92; 01.06.1992 JP 163312/92; 01.06.1992 JP 163313/92
(71) Applicant: TSUMURA & CO., Chuo-ku, Tokyo 103 (JP)
(72) Inventor: KOJIRO, Masamichi 6-1, Izaki Chuo-ku, Fukuoka-ken 810 (JP); FUKUDA, Kazunori 9-17, Kiyomizu 3-chome, Kitakyushyu-shi Fukuoka-ken 803 (JP); MIZOGUCHI, Atsushi Room 403, GuranKushihara, Kurume-shi Fukuoka-ken 830 (JP); OKADA, Hidechika 5-1, Hoshikuma 1-chome, Fukuoka-shi Fukuoka-ken 814-01 (JP)
(74) Representative: Wächtershäuser, Günter, Prof. Dr.
(86) International application number: JP9300614
(87) International publication number: WO9323033

(57) **Abstract**

An apoptosis inducer containing baicalin or baicalein as the active ingredient and utilizable as carcinostatic agent, antiviral agent, and so forth. It can be used advantageously as a new type of safe carcinostatic or anticancer drug, because it induces apoptosis programmed even in cancer cells and hence can kill cancer cells which are believed to be immortal. It is useful also in treating various diseases caused by viral infection, because it can induce apoptosis in various virally infected tissues or cells, which have been believed to be difficult to remove, to thereby permit easy removal of these tissues or cells.

## Description

### Field of Technology

The present invention relates to an apoptosis inducer which can be used as a drug such as carcinostatic agent, antiviral agent, and the like.

### Technological Background

In recent years, the manner of cell death called apoptosis (programed cell death or cell suicide) is attracting an attention. Differently from necrosis which is pathologic death of cells, in the case of apoptosis the cause of cell death is considered to be incorporated in the gene of the cells from the beginning.

Specifically, the mechanism is considered such that genes programing the apoptosis are activated due to certain external or internal factors, so-called "suicide proteins" are produced by the genes, and the cells are decomposed to death by these suicide proteins.

If the apoptosis can be induced in desired cells, it is possible to spontaneously exclude unwanted or pathogenic cells from organisms. This bears deep significance.

Glucocorticoide is the only currently known compound which can induce apoptosis in cells. Development of a compound exhibiting a more excellent apoptosis inducing action has been desired.

### Disclosure of the Invention

The present inventors have undertaken screening especially on various crude drug components in order to find compounds having an apoptosis inducing action, and have found that baicalin and baicalein exhibit excellent apoptosis inducing action and, further, that Sho-saiko-to which contains baicalin and baicalein in a large amount also exhibits the same action. These findings have led to the completion of the present invention.

Accordingly, an object of the present invention is to provide an apoptosis inducer comprising baicalin or baicalein as an effective ingredient.

Another object of the present invention is to provide a method of inducing apoptosis in organisms comprising administering an effective amount of baicalin or baicalein.

Still another object of the present invention is to provide the use of baicalin or baicalein for preparing an apoptosis inducer.

### Brief Description of the Drawings

Figure 1 is photographs of agarose gel electrophoresis showing DNA of MT4 cell line fragmented by the addition of baicalin, baicalein and Sho-saiko-to;
Figure 2 is a photograph showing morphological features of KIM-1 cells after the addition of Sho-saiko-to; and
Figure 3 is a photograph of agarose gel electrophoresis showing DNA of KIM-1 cells fragmented by the addition of Sho-saiko-to.

### Best Mode for Carrying out the Invention

Baicalin and baicalein used as effective ingredients in the present invention are compounds shown by the following chemical formula, wherein a compound having GlcA for R is baicalin and having H for R is baicalein. 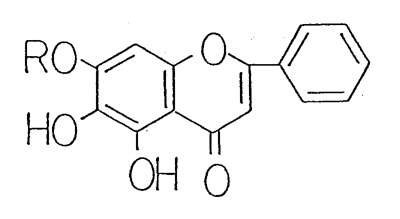
Baicalin and baicalein are already known compounds and are known as possessing anti-inflammatory action, anti-allergic action, and anti-hypertensive action. They are also known to inhibit actions of transcriptases (Japanese Patent Laid-open (Kokai) No. 163120/1989). However, the apoptosis inducing action and the effects of removing unwanted or pathogenic cells of these compounds, which are the subjects of the present invention, have not been known at all.

Any baicalin and baicalein, including those extracted from crude drugs or the like, those chemically synthesized, and those produced by microorganisms or the like, can be used as the effective ingredient.

Scutellaria Root, midnight horror (Oyoxylum indicum), and the like are given as examples of crude drugs containing a large amount of baicalin and baicalein. Although it is possible to use these crude drugs or their extracts in the present invention, Chinese medicines in which these crude drugs are formulated, such as Sho-saiko-to, Otsuji-to, Dai-saiko-to, Saiko-keishi-to, Saiko-keishi-kankyo-to, Saiko-ka-ryukotsu-borei-to, Hange-shashin-to, Oren-gedoku-to, Hange-koboku-to, Keigai-rengyo-to, Juncho-to, Gorin-san, Unsei-in, Seijo-bofu-to, Bofu-tsusho-san, Nyoshin-san, Saikan-to, Ryutan-shakan-to, Saiko-seikan-to, Nijutsu-to, Seihai-to, Saiboku-to, Shin'i-seihai-to, Sho-saiko-to-ka-kikyo-sekko, Seishin-renshi-in, San'o-shashin-to, Sairei-to, and Sammotsu-ogon-to, may be used.

Among these, Sho-saiko-to is a Chinese medicine containing crude drugs, such as Bupleurum Root, Pinellia Tuber, Scutellaria Root, Jujube Fruit, Ginseng Root, Glycyrhiza Root, and Ginger Rhizome, and is useful for the therapy of various chronic hepatitis due to its anti-inflammatory effect, cell membrane stabilizing effect, anti-allergic effect, and immunoactivation effect. It can be used with advantage for inducing apoptosis in the present invention.

Although there are some differences among various formulations of Sho-saiko-to, a typical formulation is as follows.

| | |
|---|---|
| Bupleurum Root | 4.0-7.0 |
| Pinellia Tuber | 4.0-5.0 |
| Scutellaria Root | 3.0 |
| Jujube Fruit | 2.0-3.0 |
| Ginseng Root | 2.0-3.0 |
| Glycyrhiza Root | 2.0 |
| Ginger Rhizome | 1.0-4.0 |

As the extracts of Sho-saiko-to, although extracts with various aqueous solvents are given, extracts with water are preferably used.

A specific example of the method of preparing an extract of Sho-saiko-to is extracting Sho-saiko-to with hot water of an amount of 10 times of the Sho-saiko-to and subjecting the extract obtained to filtration. If necessary, this extract may be dried to prepare a dry powder.

The apoptosis inducer of the present invention can be prepared by formulating baicalin or baicalein, as is, and known pharmaceutical carriers, and making it into preparations for oral administration, such as tablets, powders, granules, or liquids; preparations for non-oral administration, such as injections or dripping solutions; or suppositories.

When Sho-saiko-to is used as the source of baicalin or baicalein, the Sho-saiko-to of the above formulation as is or a combination of the extract with known pharmaceutical carriers is made into preparations for oral administration, such as tablets, powders, granules, or liquids; preparations for non-oral administration, such as injections or dripping solutions; or suppositories.

Carriers can be suitably selected depending on the manner in which the apoptosis inducer is administered or the type of the preparation. For example, in the case of preparations for oral administration, starch, lactose, saccharose, mannitol, carboxymethyl cellulose, corn starch, inorganic salts, and the like are used. Further, binders, disintegrators, surfactants, lubricants, fluidity enhancers, sweeteners, colorants, perfumes, and the like may be incorporated in the preparations for oral administration. Specific examples of these additives are as follows.

### (Binders)

Starch, dextrin, gum arabic powder, gelatin, hydroxypropyl starch, methyl cellulose, sodium carboxymethyl cellulose, hydroxypropyl cellulose, crystalline cellulose, ethyl cellulose, polyvinylpyrrolidone, macrogol, and the like.

### (Disintegrators)

Starch, hydroxypropyl starch, sodium carboxymethyl cellulose, calcium carboxymethyl cellulose, carboxymethyl cellulose, low-substituted hydroxypropyl cellulose, and the like.

### (Surfactants)

Sodium lauryl sulfate, soybean lecithin, sucrose fatty acid ester, polysorbate 80, and the like.

### (Lubricants)

Talc, waxes, hydrogenated vegetable oils, sucrose fatty acid ester, magnesium stearate, calcium stearate, aluminum stearate, polyethylene glycol, and the like.

### (Fluidity enhancers)

Light silicic acid anhydride, dry aluminum hydroxide gel, synthetic aluminum silicate, magnesium silicate, and the like.

Furthermore, the apoptosis inducer of the present invention may be administered as a liquid preparation for oral administration, such as suspension, emulsion, syrup, elixir, or the like. These preparations may contain sweeteners, corrigants, colorants, and the like.

On the other hand, preparations for non-oral administration are prepared by a conventional method by dissolving or suspending baicalin, baicalein, Sho-saiko-to, or an extract of these, which are effective ingredients, in distilled water for injection, a physiological saline solution, a glucose aqueous solution, vegetable oil for injection, sesame oil, peanut oil, soybean oil, corn oil, propylene glycol, polyethylene glycol, or the like as a diluent, and optionally by adding an antibacterial agent, a preservative, a stabilizer, an isotonic agent, a soothing agent, and the like.

A dose of the apoptosis inducer is normally about 100 mg to 6 g (as baicalin or baicalein) per day for an adult, administered at one time or in portions two or three times a day, in the case of a preparation for oral administration, although the dose varies depending on the route of administration, the age, and the symptoms of the patient.

The preparation for non-oral administration may be dosed in an amount of about 1-100 mg (as baicalin or baicalein) per day.

In the case where Sho-saiko-to is used as a baicalin or baicalein source, a dose of about 1-10 g of Sho-saiko-to (as Sho-saiko-to dry extract) per day for an adult is administered at one time or in portions two or three times a day.

Baicalin and baicalein used in the present invention are extremely safe substances as evidenced by the fact that no fatal incidence is observed when administered orally to mice or rats, for example, at a dose of 1 g/kg, which is the maximal administrable dose to these animals. Thus, it can be dosed without giving any concern about safety.

Because Sho-saiko-to has a long history as a Chinese medicine and its safety has been confirmed, it can be used with relief. It is extremely safe medicine as evidenced by the fact that no fatal incidence is observed when administered orally to mice or rats, for example, at a dose of 15 g/kg, which is the maximal administrable dose to these animals.

### Examples

The present invention will be hereinafter illustrated in more detail by way of examples, which shall not be construed as limiting the present invention.

### Test Example 1

### 〈Study on DNA fragmentation action in virally infected cells〉

### (Test Method)

DNA fragmentation actions of baicalin, baicalein, and Sho-saiko-to were studied by the following method.

20 ml of virally infected cells, MT4, prepared to a concentration of 1 x 10⁶ cells/ml of 10% FCS-RPMI1640 medium was placed in Falcon 3084 flasks (75 cm²). To these flasks were added baicalin or baicalein solutions in DMSO at concentrations of 4 mg/ml, 400 µg/ml, and 40 µg/ml, and Sho-saiko-to solutions in DMSO at concentrations of 200 mg/ml, 100 mg/ml, and 40 mg/ml, each in an amount of 100 µl.

After incubation at 37°C for one hour in a CO₂ incubator, 10 ml of the mixture was transferred to a centrifugal tube and centrifuged at 1,000 rpm for six minutes. After the centrifuge, the supernatant was discarded and precipitated cells were recovered. The remaining 10 ml of the mixture was incubated for a further two hours and cells were recovered by centrifuge in the same manner as above (incubation time: 3 hours).

The precipitated cells were immediately frozen each time as they were recovered, and stored at -80°C until the time when DNA was extracted.

Extraction of DNA was carried out using IsoQuick (trademark, sold by Tanehashi Co.) according to the following method.

Frozen precipitated cells were suspended in 100 µl of reagent-A, and 100 µl of reagent-1 (Lysis solution) was added to the suspension to dissolve the cells. To the solution were added 700 µl of reagent-2 (extraction matrix) and 400 µl of reagent-3 (extraction buffer), and the mixture was stirred with a vortex stirrer.

This mixture was centrifuged at 15,000 rpm for 5 minutes and the aqueous layer was recovered. One fourth amount of reagent-4 (sodium acetate solution) was added to the aqueous layer. After the addition of the same volume of isopropyl alcohol, the mixture was gently stirred to obtain DNA precipitate. The mixed solution in which the DNA was precipitated was centrifuged at 15,000 rpm for 10 minutes. After removal of the supernatant, the DNA residue was washed by centrifuge using 70% ethanol.

DNA thus obtained was dried and dissolved in 20 µl of TE buffer and the solution was subjected to agarose gel electrophoresis. The electrophoresis was carried out at 100 V for 50 minutes using 3% gel of NuSive agarose and φ x 174/Hae III digestion as a marker.

### (Results)

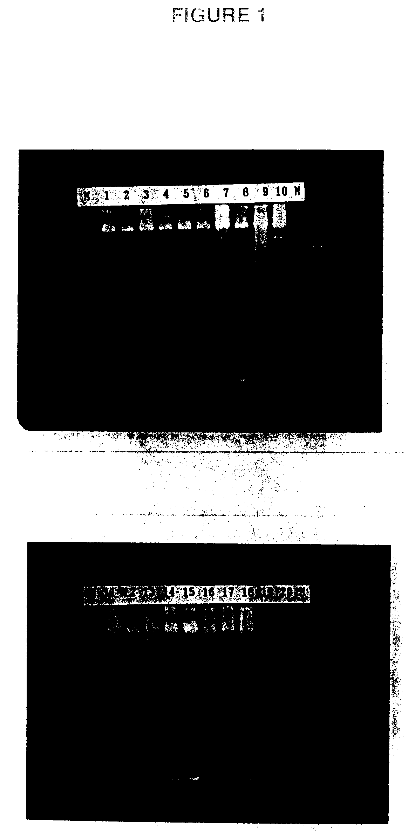

The results of electrophoresis are shown in Figure 1 (photographs), wherein lanes 1-6 in the upper photograph show DNA fragmentation when Sho-saiko-to was acted (lane 1: 200 µg/ml for 1 hour; lane 2: 500 µg/ml for 1 hour; lane 3: 1,000 µg/ml for 1 hour; lane 4: 200 µg/ml for 3 hours; lane 5: 500 µg/ml for 3 hours; lane 6: 1,000 µg/ml for 3 hours), and lanes 7-12 show DNA fragmentation when baicalein was acted (lane 7: 0.2 µg/ml for 1 hour; lane 8: 2 µg/ml for 1 hour; lane 9: 20 µg/ml for 1 hour; lane 10: 0.2 µg/ml for 3 hours; lane 11: 2 µg/ml for 3 hours; lane 12: 20 µg/ml for 3 hours).

Lanes 13-18 in the lower photograph show DNA fragmentation when baicalin was acted (lane 13: 0.2 µg/ml for 1 hour; lane 14: 2 µg/ml for 1 hour; lane 15: 20 µg/ml for 1 hour; lane 16: 0.2 µg/ml for 3 hours; lane 17: 2 µg/ml for 3 hours; lane 18: 20 µg/ml for 3 hours), and lanes 19 and 20 show DNA fragmentation of the control.

As clear from these results, DNA of viral infected cells MT4 (T-cell derived HTLV infected cell strain) were fragmented by baicalein or baicalin at a length of 200 bp or its multimers, showing that apoptosis was induced. In particular, baicalin induced apoptosis even at a low concentration of 0.2 µg/ml. The DNA was also fragmented by Sho-saiko-to at a length of 200 bp or its integral multimers, also indicating that apoptosis was induced.

### Test Example 2

### 〈Study on apoptosis induction by Sho-saiko-to〉

### (1) Preparation of Sho-saiko-to added media

Dulbecco's modified Eagle's medium supplemented with 5% fetal bovine serum, penicillin (100 U/ml) and streptomycin (100 µg/ml) was used as a base medium for the cell culture. A powder of Sho-saiko-to extract (TJ-9, manufactured by TSUMURA & Co.) was added to the medium to a concentration of 10 mg/ml and insoluble components were removed from the extract solution by centrifuge. The supernatant thus obtained was used for the experiment.

This concentrated medium was diluted to various concentrations with the above-mentioned base medium. The diluted media, either added with Sho-saiko-to or not, had an osmotic pressure and a pH within the physiological range.

### (2) Study on morphological changes of cells

5 x 10⁵ cells of KIM-1 cell line (hepatoma) and KMC-1 cell line (hepatic cholangiocarcinoma) were inoculated in T-75 flasks, and incubated in the base medium for 24 hours, whereupon the medium was replaced by a medium to which Sho-saiko-to was added at a concentration of 400 µg/ml. After 48 hours, the morphological changes of the cells were studied. Specifically, the floating cells were collected, caused to be adhered to a slide by using a cytospin, fixed with 70% alcohol, and stained by the HE staining method, following which the morphological features of the cells were observed by optical microscope.

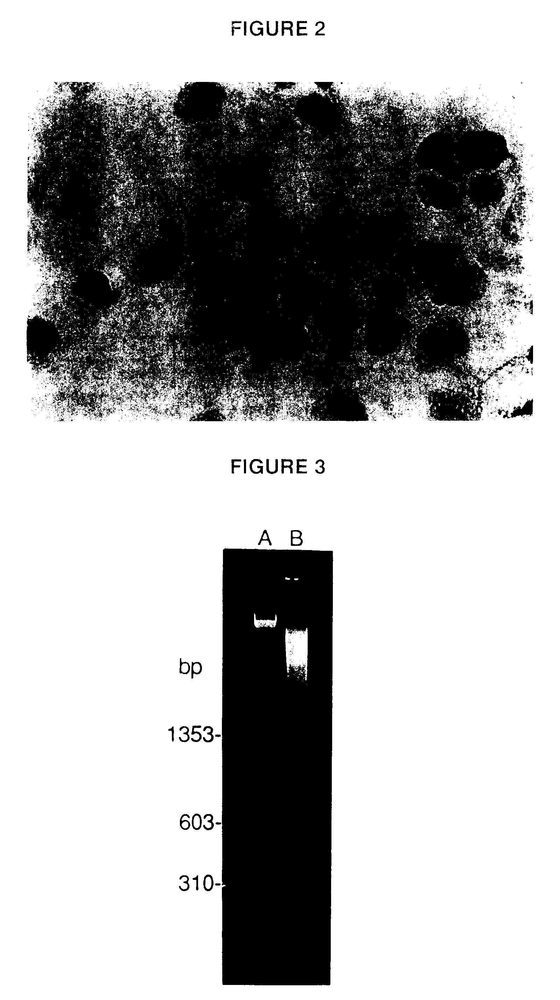

The morphological feature (a photograph) of KIM-1 cells is shown in Figure 2.

As clear from this Figure, cell shrinkage, nucleus concentration, and the like were found to occur in KIM-1 cells in the presence of Sho-saiko-to.

### (3) Study on DNA fragmentation

The floated cells and the adherent cells, collected by a scraper, both obtained in (2) above, were combined and washed twice with cold PBS. 10 mM Tris buffer solution containing 0.1 M EDTA, 0.5% SDS, and 20 µg/ml RNase (Sigma Co.) was added in an amount of 0.5 ml/10⁶ cells and the mixture was incubated at 37°C for one hours. Further, Protease K (Sigma Co.) was added to a final concentration of 100 µg/ml and the mixture was incubated at 50°C for 3 hours to obtain a cell homogenate. Proteins were removed from the cell homogenate by phenol-chloroform and DNA was recovered by ethanol precipitation. The DNA was dissolved in T10E1 (10 mM Tris-HCl/1 mM EDTA) and 10 µg of the DNA was analyzed by electrophoresis in a 1.6% agarose gel containing 0.5 µg/ml ethydium bromide.

Figure 3 shows a photograph of agarose gel electrophoresis demonstrating the DNA of KIM-1 cells obtained from control culture (lane A) and Sho-saiko-to treated culture (lane B).

As can be seen from these results, DNA fragments with a length of 180 bp or its multimers were found in the case where Sho-saiko-to was acted.

### (4) Results

Cell shrinkage, chromatin condensation, nucleus concentration, cell fragmentation, and the like are known as phenomena characteristic to apoptosis. Further, the fragmentation is known to cut DNA into oligonucleosome with a length of 180 bp or its multimers.

All results obtained in (2) and (3) above show that apoptosis was induced, thus evidencing the effect of Sho-saiko-to on induction of apoptosis.

### Example 1

### 〈Preparation of Sho-saiko-to extract (1)〉

240 g of distilled water was added to a crude drug mixture consisting of 7 g of Bupleurum Root, 3 g of Scutellaria Root, 2 g of Glycyrhiza Root, 3 g of Ginseng Root, 1 g of Ginger Rhizome, 3 g of Jujube Fruit, and 5 g of Pinellia Tuber (Sho-saiko-to; 24 g). After extraction with heating at 100°C for one hour, the extract was filtered and spray-dried to obtain 2.3 g of dry powder extract.

### Example 2

### 〈Preparation of Sho-saiko-to extract (2)〉

300 g of distilled water was added to a crude drug mixture consisting of 7 g of Bupleurum Root, 3 g of Scutellaria Root, 2 g of Glycyrhiza Root, 3 g of Ginseng Root, 1 g of Ginger Rhizome, 3 g of Jujube Fruit, and 5 g of Pinellia Tuber (Sho-saiko-to; 24 g). After extraction at 100°C for 60 minutes, the solid and liquid were separated by centrifuge. The liquid was spray-dried at a temperature below 50°C to obtain 4.5 g of dry powder extract.

The main components contained in the dry powder extract were glycyrrhizin, 42.5 mg; baicalin, 160 mg; and Saikosaponin b, 4.5 mg.

### Example 3

### 〈Preparation of Sho-saiko-to extract (3)〉

24 l of distilled water was added to a crude drug mixture consisting of 700 g of Bupleurum Root, 300 g of Scutellaria Root, 200 g of Glycyrhiza Root, 300 g of Ginseng Root, 100 g of Ginger Rhizome, 300 g of Jujube Fruit, and 500 g of Pinellia Tuber (Sho-saiko-to; 2.4 kg). The mixture was heated and, when the temperature went up to 100°C, extracted for one hour. The extract was centrifuged to remove residue, thus obtaining 20 l of a solution.

This solution was filtered through 0.3 µm membrane filter (Toyo Filter Co.) for sterilization and clarification, and the filtrate was subjected to ultra-filtration using Diafilter G-10T (Bioengineering Co., fractional molecular weight: 10,000). The ultra-filtration was carried out by setting a membrane with a diameter of 152 mm in the bottom of a container with an internal volume of 2.0 l at a pressure of 3 kg/cm², while adding about 2 l of distilled water as the liquid in the container was concentrated.

As a result, 20 l of ultra-filtrate was obtained.

### Example 4

### 〈Preparation of granules (1)〉

10 g of baicalin, 89 g of lactose, and 1 g of magnesium stearate were mixed, and the mixture was made into tablets using a single tablet castor to obtain slug tablets, each having a diameter of 20 mm and a weight of about 2.3 g. The slug tablets were pulverized using an oscillator, granulated, and sieved to obtain 20-50 mesh granules. (The granules contained 100 mg of baicalin in 1 g. A dose of 1-2.5 g/adult is to be administered in portions several times a day.)

### Example 5

### 〈Preparation of granules (2)〉

200 g of the dry powder extract of Sho-saiko-to prepared in Example 1, 89 g of lactose, and 1 g of magnesium stearate were mixed, and the mixture was made into tablets using a single tablet castor to obtain slug tablets, each having a diameter of 20 mm and a weight of about 2.3 g. The slug tablets were pulverized using an oscillator, granulated, and sieved to obtain 20-50 mesh granules.

### Example 6

### 〈Preparation of tablets (1)〉

2.5 g of baicalin, 20 g of microcrystalline cellulose, and 5 g of magnesium stearate were mixed, and the mixture was made into tablets using a single tablet castor to obtain tablets, each having a diameter of 7 mm and a weight of about 225 mg. Each tablet contained 20 mg of baicalin. (A daily dose of 5-7 tablets to adult is to be administered in portions several times a day.)

### Example 7

### 〈Preparation of tablets (2)〉

200 mg of the dry powder extract of Sho-saiko-to prepared in Example 2, 20 g of microcrystalline cellulose, and 5 g of magnesium stearate were mixed, and the mixture was made into tablets using a single tablet castor to obtain tablets, each having a diameter of 7 mm and a weight of about 225 mg. Each tablet contained 200 mg of the dry powder extract of Sho-saiko-to.

### Example 8

### 〈Preparation of capsules (1)〉

10 g of baicalein, 89.5 g of corn starch, and 0.5 g of light silicic acid anhydride were homogeneously mixed. 200 mg of the mixture was filled in each capsule to prepare capsules. (Each capsule contained 20 mg of baicalein. A daily dose of 5-7 capsules to adult is to be administered in portions several times a day.)

### Example 9

### 〈Preparation of capsules (2)〉

500 mg of the dry powder extract of Sho-saiko-to prepared in Example 2 was filled in each hard capsule to prepare capsules.

### Example 10

### 〈Preparation of injection preparation (1)〉

100 mg of glucose and 10 mg of baicalin were dissolved in an appropriate amount of distilled water for injection. The total volume was made 15 ml by further adding distilled water. 5 ml of the solution was filled in each ample and sterilized with heating at 121°C for 15 minutes, thus obtaining an injection preparation.

### Example 11

### 〈Preparation of injection preparation (2)〉

300 g of alanine (containing no exothermic components) was added to 20 l of the ultra-filtrate obtained in Example 3 and dissolved. The mixture was lyophilized and the lyophilized product was filled in 900 vials, thus obtaining an injection preparation. Each vial contained 406 mg of lyophilized substance, which could be readily dissolved in 10 ml of purified water. The injection solution after dissolution had a transparency of 92% (at 550 nm), complying with the exothermic substance test according to The Pharmacopoeia of Japan.

### Industrial Applicability

The apoptosis inducer containing baicalin or baicalein as the effective ingredient of the present invention can effectively induce apoptosis in organisms. Differently from the cell death due to necrosis, apoptosis is the cell death of which the cause have been incorporated in the cells from the beginning. Because of this, it is possible to exclude unwanted or pathogenic cells from organisms in a natural manner.

Conventionally, medicines, such as alkylation agents, antimetabolites, antibiotics, DNA synthesis inhibitors, and immunopotentiators, have been used as carcinostatic agents or an anticancer drugs. Among these medicines, alkylation agents, antimetabolites, antibiotics, and DNA synthesis inhibitors affect not only cancer cells but also normal cells and their side effects are too strong. Immunopotentiators have a problem in that their actions are weak.

However, if the apoptosis inducer of the present invention is used, it is possible to induce apoptosis in cancer cells and kill the cancer cells which are believed to be immortal. Thus, it can be used advantageously as a new type of safe carcinostatic or anticancer agent.

It is useful also in treating various diseases caused by viral infection, because it can induce apoptosis in various virally infected cells, which have been believed to be difficult to remove, thereby permitting easy removal of these cells.

## Claims

1. An apoptosis inducer comprising baicalin or baicalein as an effective ingredient.

2. The apoptosis inducer as claimed in claim 1, wherein Sho-saiko-to is used as the source of baicalin or baicalein.

3. A composition for inducing apoptosis comprising effective amounts of baicalin or baicalein and pharmaceutically acceptable carriers.

4. The composition for inducing apoptosis as claimed in claim 3, wherein Sho-saiko-to is used as the source of baicalin or baicalein.

5. A method of inducing apoptosis in organisms comprising administering an effective amount of baicalin or baicalein.

6. The method of inducing apoptosis as claimed in claim 5, wherein Sho-saiko-to is used as the source of baicalin or baicalein.

7. Use of baicalin or baicalein for preparing an apoptosis inducer.

8. The use as claimed in claim 7, wherein Sho-saiko-to is the source of baicalin or baicalein.
